# EUROPEAN PATENT SPECIFICATION

(11) **EP 0 325 605 B1**
(45) Date of publication and mention of the grant of the patent: **23.03.1994**
(21) Application number: 87906593.6
(22) Date of filing: 08.10.1987
(51) Int. Cl.: A61B 5/04, A61B 5/03, A61B 5/0448

(54) **INTRAUTERINE PROBE**
GEBÄRMUTTERSONDE
SONDE INTRA-UTERINE

(30) Priority: 08.10.1986 GB 8624169
(43) Date of publication of application: 02.08.1989
(73) Proprietor: IMPERIAL COLLEGE OF SCIENCE, TECHNOLOGY & MEDICINE, London SW7 2BB (GB)
(72) Inventor: SUTHERLAND, Ian, Alexander, Harpenden, Hertfordshire AL5 2Q2 (GB); RANDALL, Nigel, John, London NW6 6TS (GB); STEER, Philip, James, Kingston Upon Thames Surrey KT2 7BJ (GB)
(74) Representative: Woodcraft, David Charles
(86) International application number: GB8700713
(87) International publication number: WO8802616

(56) References cited:
- EP-A- 0 092 982
- FR-A- 2 020 437
- GB-A- 2 016 706
- US-A- 3 326 207
- US-A- 3 545 432

## Description

This invention relates to an intrauterine probe which is suitable for use in monitoring conditions during labour, i.e. the condition of the fetus and also the condition of the mother.

The desirability of monitoring fetal heart rate (FHR) and intrauterine pressure (IUP) during a difficult labour is well known. In practice, IUP has been measured using a pressure catheter, and a separate device has been used to monitor FHR. Generally, FHR has been monitored by recording the voltage between two electrodes of which one is in the form of a body clip and the other (the "indifferent" or "reference" electrode) is spaced a short distance from the clip in contact with surrounding tissue (usually maternal).

EP-A-0092982 discloses an intrauterine probe which comprises an elongate flexible body member having a rounded distal end and having at least one electrode mounted on the outer surface of the body member for monitoring the dilation of the cervix and the maternal heart rate. The body member is anchored to a leg plate to prevent the probe from moving longitudinally with respect to the leg plate, which is anchored to a material leg by a circumferential strap. A scalp clip is attached to that part of the fetus which is presented for delivery for monitoring the fetal heart rate. A clip is necessarily invasive to the fetus, and is a disincentive to routine monitoring during labour. It is desirable clinically to carry out fetal monitoring routinely but this is unlikely to be achieved unless the procedure can be made more acceptable to women.

It would also be desirable to devise a system which would enable FHR and other factors to be monitored without the need to make separate trans-vaginal insertions.

US-A-3572322 discloses a transducer assembly which comprises an elongate flexible flattened body member having a rounded distal end and having at least one electrode mounted in one flat face thereof. The transducer assembly is adapted to be held against the surface of the patient's body.

According to the present invention there is provided an intrauterine probe for monitoring the condition of the human fetus during labour which comprises a flexible, elongate body member formed from an electrically insulating material and having a flattened cross-section and a rounded distal end; the body member having at least one electrode located at the distal end of the body member and in one face thereof; said body member having a length and a flexibility such that the probe is insertable into the cervix and capable of being guided around the fetus so as to extend along a substantial length thereof.

Preferably, the portions of insulating material surrounding the electrodes are formed from a resilient foam material having non-communicating cells, and the portions of insulating material bounding the electrodes preferably have a rounded upper surface in cross section.

The probe preferably has at least two electrodes spaced longitudinally thereof, preferably has a distal electrode, and preferably a plurality of electrodes spaced over a distance which, in use, is sufficient to encompass the fetal head and neck, e.g. a distal electrode and two or more additional electrodes located in a group, the spacing between the distal electrode and the proximal additional electrode being greater than the spacing between additional electrodes in the group. The probe may also include a pressure transducer.

A probe in accordance with the invention may be used in conjunction with a processor which is adapted to distinguish between signals representing fetal heart rate (FHR) and maternal heart rate (MHR).

We have found that consistent and reliable signal detection can be achieved using the probe according to the invention, which is at least comparable with that from a conventional fetal scalp clip electrode.

The probe is generally flat in cross-section with rounded edges and with the electrodes located in the surface of one face. The flat sides enable the easy positioning of the electrodes, while fulfilling the requirement of surrounding the electrodes with insulating material. The shape also confers probe flexibility along the surface lying against the fetus, while providing sufficient transverse rigidity to allow the clinician to have control over the direction of insertion.

Details of construction and operation of intrauterine probes in accordance with the invention will be apparent from the following description and accompanying drawings in which:-
Figure 1 is a perspective view of an embodiment of a probe in accordance with the invention,
Figure 2 is a cross-sectional view through an electrode of the probe of Figure 1,
Figure 2A is a cross-sectional view through the probe between electrodes,
Figure 3 is a schematic section of a uterus showing the probe in use,
Figure 4 is a diagrammatic exploded view illustrating one method of manufacturing the probe,
Figure 5 is an exploded view of a pressure sensor on an enlarged scale,
Figure 6 is a schematic representation of the connection of the electrodes to a signal processor,
Figure 7 is a typical trace showing the fetal and maternal heart beats.

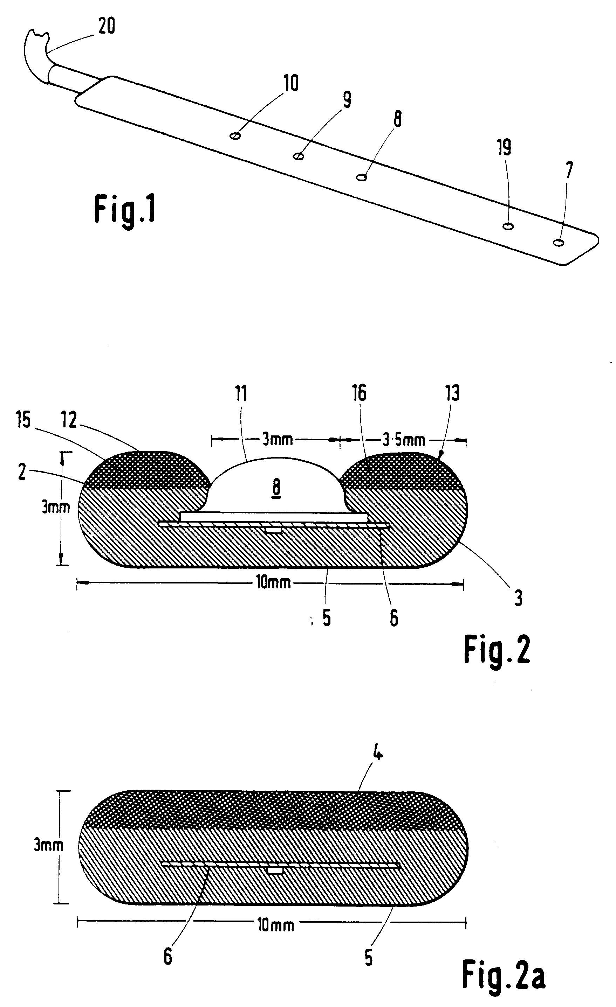

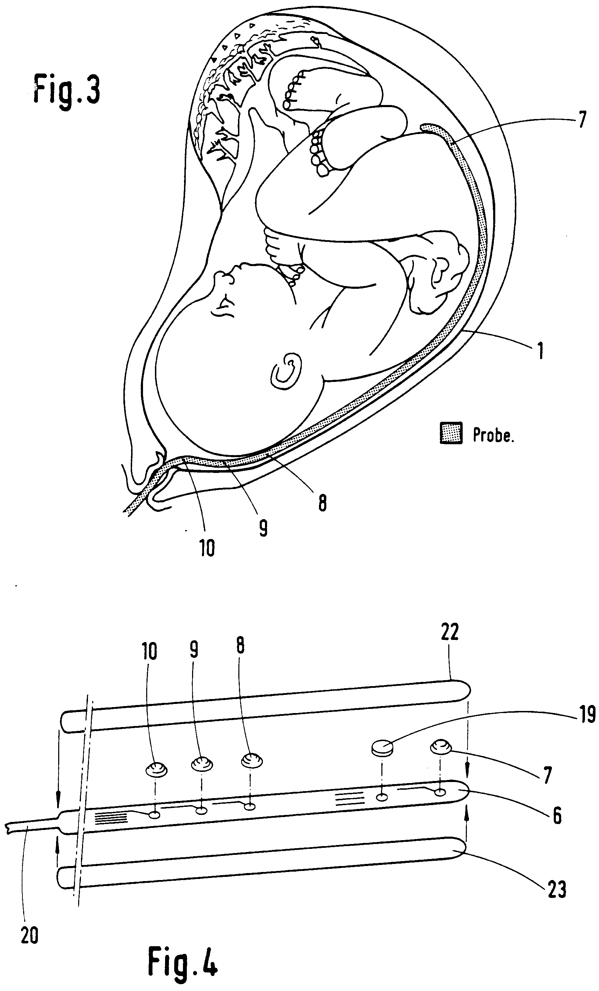

Referring to the drawings and in particular Figures 1 2 & 2A, the probe comprises an elongate body 1 about 40 to 50 cms in total length. As best seen in Figures 2 & 2A, the probe has a flattened configuration with rounded edges 2, 3 and generally flat upper and lower faces 4,5. Typically, the probe is about 1 cm wide and about 3 mm thick. A flexible printed circuit board 6 carries spaced electrodes e.g. of stainless steel 7, 8, 9 & 10 each having a domed head 11 and is encapsulated in a flexible plastics potting compound, such as a 2-part polyurethane composition. The dimensions and inherent flexibility of the plastics material are such that the probe takes up the position shown in Figure 3 when inserted in the uterus. The particular potting composition employed was a 2-part polyurethane composition obtainable from Emerson & Cumming Ltd. 866 Uxbridge Road, Hayes, Middlesex, under the trade name CPC 19 flexible polyurethane potting compound. Probes having a stiffness (Young's Modulus) in the range of 1 to 10 meganewtons per square metre are suitable.

As shown in Figure 2, the domed head 11 of the electrode 8 is effectively shrouded by rounded portions 12 & 13 of the insulating material of the body. The surface of the domed portion 11 lies substantially in the same plane as the flat face 5 of the body. In use, this ensures that amniotic fluid is squeezed out to form a thin electrolyte film between the electrode 8 and the reference electrode when the probe is suitably located in relation to the uterine or cervical walls.

As can be seen from Figure 1, the electrodes are spaced so that the distal electrode 7 is spaced from the other electrodes 8, 9 & 10. As a consequence, the distance between electrode 7 and its nearest electrode 8 is greater than the inter-electrode spacing in the group of electrodes 8, 9 & 10. Because of its greater spacing the electrode 7 is generally used as the reference electrode. However, as will be described below, the signals detected by any pair of electrodes may be used for measuring the FHR. The spacing between electrode 7 and electrode 8 may typically be 8 to 12 cms, while the inter-electrode spacing in the group of electrodes 8, 9 & 10 may be, for example, 3 to 6 cms. As the birth progresses, the signals detected by each electrode may vary in strength and the signals may be processed by selecting, at any one time, the outputs from the pair of electrodes which give the best signal.

In contrast with the experience of scalp-clip monitors, the quality of the signals obtained from the probes of the invention often improve as the birth progesses. This is believed to be because the probe is pressed more firmly against the baby's back and head as the fetal head passes into the birth canal.

Referring again to Figure 2, the portion of insulating material surrounding the electrodes may be formed from a unicellular or closed cell foam. These portions 15 & 16 are shown in cross hatching in Figures 2 & 2A. This may enable the probe to be pressed less tightly against the fetal skin while still minimising the effective 'electrolyte' film thickness.

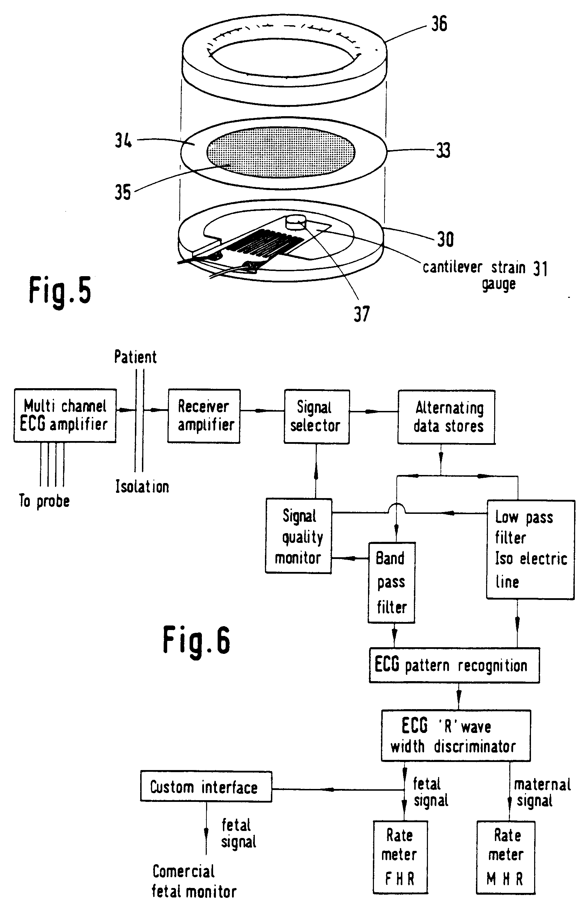

Manufacture of the probe is illustrated in Figure 4. The electrodes 7, 8, 9 & 10 and also a pressure transducer 19 are attached to a printed circuit board 6. Board 6 includes conductor strips linking the electrodes to a multifilament cable 20 which is connected to a processor (see Figure 6). Conveniently, the processor may include a digital display unit but may incorporate an oscilloscope and a chart recorder (not shown). Circuit board 6 and attached electrodes are encapsulated in a potting compound to form a shaped probe body having the configuration shown in Figures 1, 2 & 2A by moulding between upper and lower moulds 22 & 23. A foaming agent may be introduced into the potting composition, or into a portion which will form the parts 16 & 17 of the probe body (see Figure 2).

The minimum number of electrodes in the group 8, 9 & 10 is one but the more electrodes are present, the better the chance of maintaining good signal quality during birth. Generally 2 to 4 electrodes in the group are usually satisfactory. The group of electrodes are preferably spaced over a distance sufficient to encompass the fetal head and neck, e.g. at least about 5 cms, typically 5 to 15 cms. Interelectrode spacing is generally less than the distance between the electrode nearest the tip, i.e. electrode 8, and the distal electrode 7. This distance is commonly about 15 to 20 cms, e.g. about 18 cms. At present, the preferred configuration is a distal electrode 7 and three equally-spaced additional electrodes 8, 9 & 10. The interelectrode spacing being about 5 cms and the spacing from the distal electrode 18 cms.

In use, therefore, one at least of the group of electrodes 8, 9 & 10 is to a large extent redundant. At least one of the electrodes in the group will be useful, depending to some extent on the length of the fetus, and fetal movement after insertion. Sometimes two of the electrodes in the group will be utilised.

A probe of the invention can be used from the time at which the cervix is dilated to, say, 1 cm. The probe is inserted around the head or neck of the fetus and towards its lower trunk, and the flattened shape ensures that one face is stably oriented in contact with at least the head of the fetus. The intention is that the probe should be inserted to the extent that the distal electrode is on or adjacent to the lower trunk of the fetus, while one at least of the group of electrodes is in good contact with the head or neck of the fetus (see Figure 3). At a later stage of labour to that shown in Figure 3, the fetus' head and neck will be pressed against the electrodes 8, 9 & 10.

A probe of the invention in its preferred form includes a pressure sensor specifically to measure Intrauterine amniotic fluid Pressure (IUP). The location of this sensor is such that the point at which IUP is measured is both reasonably well known and unlikely to be influenced by unknown causes. By contrast with a pressure catheter, a pressure transducer and other sensors operating as miniature load cells (force sensors) for use in the invention can be constructed very cheaply.

Preferably the IUP sensor 19 is located at or close to the distal end of the probe. Its internal construction is shown in Figure 5 and comprises a base carrier 30 which may be manufactured in metal (e.g. stainless steel), but is preferably moulded from plastics material (e.g. ABS plastic) and supporting a cantilever strain gauge (sensor) 31. The output from the strain sensor is connected to the printed circuit board 6. Overlying the base carrier is an assembly 33 comprising an annular thin film membrane 34 which supports a rigid plastics disc 35. The pressure sensor is completed by a sealing ring 36, which may be moulded in a rigid plastic e.g. ABS, or in a soft rubber-like material; over the base carrier and may include a grid structure to protect the membrane. In use, variations of IUP will cause the disc 35 to move inwardly and outwardly, thus applying more or less force to the strain gauge 31 via a contact button 37.

A probe of the invention provides a single device for the measurement of those criteria which are presently considered to be important for the good health of the mother and her baby. The probe can also be used, without changing its essential function, to measure further or different parameters; for example, there may be a temperature sensor which could be used to detect maternal hyperthermia or temperature variation during contraction, and/or an optical sensor which could be used to detect, say, meconium and/or fetal blood oxygen levels when used as a trans-cutaneous oximeter.

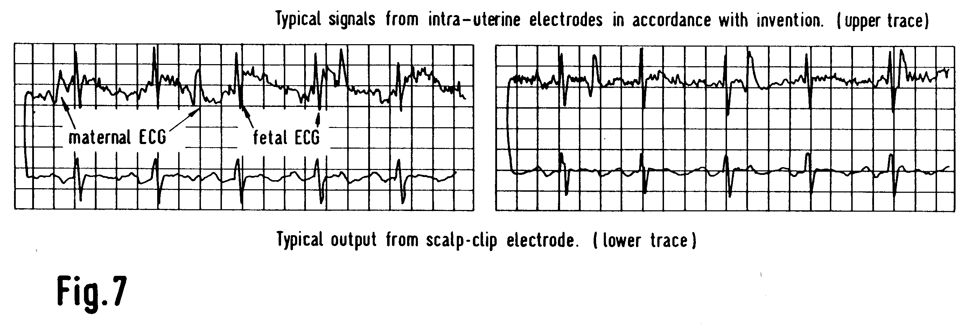

For use, a probe of the invention is in connection with a processor and display means. An arrangement for processing and displaying the output from the probe is shown in Figure 6. Typical traces produced by a chart recorder connected to a probe in accordance with the invention are shown in Figure 7. The output from the electrode pairs is a mixture of maternal and fetal heart rates and background 'noise' deriving from other muscular activity. The specific processing to distinguish the maternal and fetal ECG complexes takes advantage of the differing morphologies of each. Results with the invention have demonstrated that the relative amplitudes of the fetal and maternal complexes during a given labour are unpredictable but the measured width of the fetal complex is consistently less than that of the maternal complex during the same labour. Hence, either frequency domain pattern recognition of the spectral components (amplitude and/or phase) after Fourier transformation of each complex, or temporal/spatial pattern recognition in real time and/or by retrospective analysis can be applied. Although the fetal heart rate signal cannot always be recognized uniquely by the measured width of its 'R' component, a combination of R wave width recognition in conjunction with comparison with a stored pattern can be used to separate unambiguously the fetal and maternal heart rates from each other and from background noise. In this way, those signals recorded by the electrodes processed in order to provide separate displays of FHR and MHR, together with the data obtained from the IUP sensor and other sensors at least. The display is visual, e.g. on a screen, but for the purposes of record a chart recorder will be used (no other display may be necessary). Alternatively, the processed fetal heart signal and IUP can be made compatible with current commercial fetal monitors from which the FHR and IUP can be presented in the normal way.

Figure 6 shows an arrangement in accordance with the invention for processing and displaying the signals detected by the probe.

Signals from the electrodes are fed to a multi-channel ECG amplifier and the amplifier output connected to the processing equipment via a patient isolation link such as a fibre optic cable. The transmitted signals are re-amplified and then passed to a signal selector which monitors the signals and selects the best signals from any pair of electrodes. The selected signal is passed via a data store to a band width filter and a low pass filter (to establish the isoelectric line for the wave form). A feed-back to the signal selector is provided via a signal quality monitor to enable the signal selector to select signals on the basis of quality of fetal heart signal content as well as signal strength. After processing by an EGG pattern recognition unit, the signals are separated by an ECG R wave width discriminator into the fetal and maternal signals and the outputs displayed on rate meter display units, such as digital display units.

## Claims

1. An intrauterine probe for monitoring the condition of the human fetus during labour which comprises a flexible, elongate body member (1) formed from an electrically insulating material and having a flattened cross-section and a rounded distal end; the body member (1) having at least one electrode (7, 8, 9, 10, 19) located at the distal end of the body member and in one face thereof; said body member having a length and a flexibility such that the probe is insertable into the cervix and capable of being guided around the fetus so as to extend along a substantial length thereof.

2. A probe according to claim 1 wherein the body member has a length of about 40 to 50 cms.

3. A probe as claimed in claim 1 or 2 wherein a plurality of electrodes are located in the body member, the electrodes being spaced lengthwise of the probe.

4. A probe as claimed in claim 3 wherein an electrode is located at the distal end of the body member and two or more additional electrodes are spaced from said distal end lengthwise of the probe.

5. A probe as claimed in claim 4 wherein the spacing between said additional electrodes is less than that between the distal electrode and the nearest additional electrode.

6. A probe as claimed in any one of the preceding claims wherein the surface of each electrode lies substantially in the plane of the face of the body member whereby in use, amniotic fluid is squeezed out to form a thin electrolyte film of high impedance between electrodes.

7. A probe as claimed in any one of the preceding claims wherein the or each electrode is connected to a printed circuit board (6) which is encapsulated in a flexible plastics potting compound so that each electrode is surrounded by said potting compound.

8. Apparatus for monitoring fetal heart rate which comprises a probe as claimed in any one of the preceding claims connected to a processor and to display means, wherein signals from the or each electrode are processed and displayed on said display means.

9. Apparatus as claimed in claim 8 wherein the processor is adapted to process the signals to distinguish maternal and fetal heart rates (MHR and FHR) by R wave width recognition in conjunction with a stored pattern and to provide separate displays of FHR and MHR.

10. Apparatus as claimed in claim 8 or 9 wherein said display means includes data obtained from an intrauterine pressure sensor and/or temperature sensor located on the probe.

## Patentansprüche

1. Gebärmuttersonde zum Überwachen des Zustandes eines menschlichen Fötus während der Arbeit, mit einem flexiblen langen Körper (1), gebildet aus elektrisch isolierendem Material, mit abgeflachtem Querschnitt und einem gerundeten distalen Ende, wobei der Körper (1) wenigstens eine Elektrode (7, 8, 9, 10, 19) aufweist, die am distalen Ende des Körpers und auf einer Seitenfläche angeordnet ist; wobei der Körper eine solche Länge und eine solche Flexibilität hat, daß die Sonde in den Cervix einführbar und um den Fötus derart herumfuhrbar ist, daß sie sich entlang eines wesentlichen Teiles von dessen Länge erstreckt.

2. Sonde nach Anspruch 1, wobei der Körper eine Länge von etwa 40 bis 50 cm hat.

3. Sonde nach Anspruch 1 oder 2, wobei eine Mehrzahl von Elektroden am Körper angeordnet ist und die Elektroden in Längsrichtung der Sonde einen gegenseitigen Abstand aufweisen.

4. Sonde nach Anspruch 3, wobei die Elektrode am distalen Ende des Körpers angeordnet ist, und zwei oder mehrere zusätzliche Elektroden vom distalen Ende entfernt und in Längsrichtung der Sonde angeordnet sind.

5. Sonde nach Anspruch 4, wobei der Abstand zwischen den zusätzlichen Elektroden geringer als jener zwischen der distalen Elektrode und der nächsten zusätzlichen Elektrode ist.

6. Sonde nach einem der vorausgegangenen Ansprüche, wobei die Fläche einer jeden Elektrode im wesentlichen in der Ebene der Fläche des Körpers liegt, wobei während des Gebrauches amniotische Flüssigkeit ausgepreßt wird, um einen dünnen elektrolytischen Film hoher Impedanz zwischen den Elektroden zu bilden.

7. Sonde nach einem der vorausgegangenen Ansprüche, wobei die oder jede Elektrode an eine bedruckte Schaltkarte (6) angeschlossen ist, die von einer flexiblen plastischen Hüllverbindung eingehüllt ist, so daß jede Elektrode von der Hüllverbindung umgeben ist.

8. Vorrichtung zum Überwachen des fötalen Herzschlages, mit einer Sonde gemäß einem der vorausgegangenen Ansprüche, angeschlossen an einen Prozessor und an ein Display, wobei die Signale aus der oder jeder Elektrode verarbeitet und auf dem Display wiedergegeben werden.

9. Vorrichtung nach Anspruch 8, wobei der Prozessor derart gestaltet ist, daß er die Signale verarbeitet, um maternelle und fötale Herzschläge (MHR und FHR) durch R-schwingungsbreiten Erfassung in Verbindung mit einem gespeicherten Muster unterscheidet und getrennte Displays des FHR und des MHR liefert.

10. Vorrichtung nach Anspruch 8 oder 9, wobei das Display Daten aufweist, die von einem Gebärmutterdrucksensor und/oder Temperatursensor empfangen werden, der an der Sonde angeordnet ist.

## Revendications

1. Sonde intra-utérine pour surveiller l'état du foetus humain durant le travail, comprenant un membre formant corps allongé (1) souple, formé à partir d'un matériau électriquement isolant et ayant une section transversale aplatie et une extrémité distale arrondie; le membre formant corps (1) ayant au moins une électrode (7, 8, 9, 10, 19) située à l'extrémité distale du membre formant corps et dans l'une de ses faces; ledit membre formant corps ayant une longueur et une souplesse telles que la sonde peut être insérée dans le col de l'utérus et est capable d'être guidée autour du foetus, de manière à s'étendre sur une longueur substantielle de celui-ci.

2. Sonde selon la revendication 1, dans laquelle le membre formant corps a une longueur d'à peu près 40 à 50 cm.

3. Sonde selon la revendication 1 ou 2, dans laquelle une pluralité d'électrodes est située dans le membre formant corps, les électrodes étant espacées dans la direction longitudinale de la sonde.

4. Sonde selon la revendication 3, dans laquelle une électrode est située à l'extrémité distale du membre formant corps et deux ou plusieurs électrodes supplémentaires sont espacées de ladite extrémité distale, dans la direction longitudinale de la sonde.

5. Sonde selon la revendication 4, dans laquelle l'espacement entre lesdites électrodes supplémentaires est inférieur à celui entre l'électrode distale et l'électrode supplémentaire la plus proche.

6. Sonde selon l'une quelconque des revendications précédentes, dans laquelle la surface de chaque électrode se situe substantiellement dans le plan de la face du membre formant corps, de manière que, lors de l'utilisation, du liquide amniotique soit pressé pour former un mince film d'électrolyte à impédance élevée, entre les électrodes.

7. Sonde selon l'une quelconque des revendications précédentes, dans laquelle la ou chaque électrode est reliée à une plaquette de circuits imprimés (6), qui est enfermée dans un composé d'empotage en matière plastique souple, de manière que chaque électrode soit entourée par ledit composé d'empotage.

8. Appareil pour surveiller un pouls foetal, comprenant une sonde selon l'une quelconque des revendications précédentes, reliée à un processeur et à des moyens d'affichage, dans lequel les signaux provenant de la ou de chaque électrode sont traités et affichés sur lesdits moyens d'affichage.

9. Appareil selon la revendication 8, dans lequel le processeur est adapté pour traiter les signaux, en vue de distinguer les pouls maternel et foetal (MHR et FHR) par une reconnaissance de largeur d'onde R, conjointement avec un modèle stocké et de fournir des affichages séparés des pouls FHR et MHR.

10. Appareil selon la revendication 8 ou 9, dans lequel lesdits moyens d'affichage comprennent des données obtenues à partir d'un capteur de pression intrautérine et/ou d'un capteur de température intrautérine, situés sur la sonde.
